Europäisches Patentamt

European Patent Office

Office européen des brevets

(19)

(11) Publication number: **0 063 482**
A2

(12) **EUROPEAN PATENT APPLICATION**

(21) Application number: 82301961.7

(22) Date of filing: 16.04.82

(51) Int. Cl.³: **C 12 P 21/00**
**C 12 N 15/00, C 07 G 7/00**
**//C12R1/91**

(30) Priority: 17.04.81 US 255138
18.08.81 US 293775
05.03.82 US 355085

(43) Date of publication of application:
27.10.82 Bulletin 82/43

(84) Designated Contracting States:
BE DE FR GB

(71) Applicant: MELOY LABORATORIES, INC.
6715 Electronic Drive
Springfield Virginia 22151(US)

(72) Inventor: Braude, Irwin Allen, Dr.
5711 Walnut Wood Lane
Burke Virginia 22015(US)

(74) Representative: Laredo, Jack Joseph et al,
Elkington and Fife High Holborn House 52/54 High
Holborn
London, WC1V 6SH(GB)

(54) Production of immune interferon and its mRNA.

(57) Process for the production of IFN-γ comprising incubating for a period of time at least sufficient for the production of IFN-γ, a viable cell suspension which includes (a) leukocytes, (b) an inducer for production of IFN-γ, and (c) a modulator for said inducer selected from the group consisting of mezerein and 12, 13-phorbol butyrate, said inducer and said modulator being present in said suspension in amounts sufficient to stimulate production of IFN-γ; process for producing homogeneous IFN-γ; process for producing IFN-γ mRNA; and cloning process for production of IFN-. Products disclosed are homogeneous IFN-γ and its 2 species; and the host cells containing the recombinant cloning vehicle containing DNA segment coding for IFN-γ.

-1-

PRODUCTION OF IMMUNE
INTERFERON AND ITS mRNA

This invention relates to processes for producing immune interferon and its mRNA, and to homogeneous immune interferon ("IFN-$\gamma$").

More particularly, one aspect of the invention relates to a process for the production of high yields of human IFN-$\gamma$ from induced human leukocytes modulated with mezerein or 12,13-phorbol dibutyrate. Preferably, the leukocytes are induced by a calcium ionophore selected from A-23187, ionomycin, and 1,1-dimethyl-6,6,7,7,8,8,8-heptafluoro-3,5-octanedione.

Another aspect of this invention relates to IFN-$\gamma$ which has been purified to homogeneity.

A further aspect of this invention relates to a process for the recovery of IFN-$\gamma$ mRNA, and to a cloning process for the production of IFN-$\gamma$.

This application is a convention application based on our copending U.S. applications Serial No. 255,138, filed April 17, 1981, Serial No. 293,775, filed August 18, 1981 and Serial No. 355,085, filed March 5, 1982.

Interferon was discovered by Isaacs and Lindenmann in 1957, who observed that fluids from virus-infected cell cultures contained a protein which could react with normal cells to render them resistant to infection by a wide variety of viruses. In addition to potent antiviral effects, interferon possesses anticellular, immunoregulatory and antitumor activities. The use of interferon in the treatment of cancer and viral infections in animals has met with some successes, and consequently its use in man is of great interest.

Interferons are classified into three major species designated alpha or leukocyte interferon (IFN-$\alpha$), beta or fibroblast interferon (IFN-$\beta$) and gamma or immune interferon (IFN-$\gamma$). IFN-$\alpha$ and IFN-$\beta$ are induced by viruses or synthetic polynucleotides.

IFN-$\gamma$ has been induced in primed lymphocytes by a specific antigen or in unprimed lymphocytes by certain T-cell mitogenic proteins. It is believed that protein mitogens cause cell membrane receptors to form clusters which are termed "patches." Presumably, the patching event allows a rapid influx of calcium into the cell which, for a reason not entirely understood, initiates production of IFN-$\gamma$.

IFN-$\gamma$ may be distinguished from the IFN-$\alpha$ and IFN-$\beta$ species of interferon by its inability to be neutralized by antibodies directed against either IFN-$\alpha$ or IFN-$\beta$, and its ability to be neutralized by antibodies directed against IFN-$\gamma$. IFN-$\gamma$ may also be characterized by the fact that is biological activity is largely destroyed by being placed in a

0063482

low pH environment, such as pH 2, and also when brought into contact with sodium dodecyl sulfate.

Interferon related research has primarily concentrated on the IFN-α and IFN-β species. For example, considerable work on producing IFN-α has been done by Dr. Kari Cantell in Finland. See, "Production and Preparation of Human Leukocyte Interferon", K.E. Morgensen and K. Cantell, 1 Pharmac. Ther. C., 369-381, 1977. The amino acide sequences of IFN-α and IFN-β have recently been identified by protein sequence analyses and work on the sequences of their respective cloned complementary DNA. Furthermore, there is now evidence that multiple sub-species of IFN-α and IFN-β exist.

Very little is known about the structure of IFN-γ or its biological properties. It is now believed, however, that IFN-γ may be more active as a cell growth inhibitory agent, immunoregulatory agent and antitumor agent, and it may possess the ability to potentiate both the antiviral and anticellular effects of either IFN-α or IFN-β . Thus, IFN-γ may be of greater interest than IFN-α and IFN-β .

A variety of T-cell mitogenic proteins have been identified for inducing IFN- from unprimed lymphocytes. Some of the more commonly used proteins are plant lectins such as concanavalin A and phytohemagglutinin A, or other proteins known to be mitogenic such as staphylococcal and enterotoxin A and staphylococcal protein A.

An article, "Human Immune Interferon: Induction in Lymphoid Cells by a Calcium Ionophore," by F. Dianzani et al., 29 Infection and Immunity, 561-563, August 1980, described the use of the calcium ionophore A-23187 as an IFN-$\gamma$ inducer. (A-23187 is described in U.S. Patent 3,923,823.) Calcium ionophore A-23187 is not a protein. It is believed that A-23187, due to its lipophilic properties and high affinity for calcium, permits a rapid influx of calcium across the cell membrane thereby simulating the patching mechanism of the protein inducers. However, using the Dianzani et al. procedure, the IFN-$\gamma$ activity is barely perceptible; only 10-20 units per ml. of IFN-$\gamma$ activity has been observed. As used herein, a unit is equivalent to 50% inhibition of viral cytopathic effects. (Comparison of titers among different laboratories is not readily made because there is no accepted international standard for IFN-$\gamma$).

This invention teaches a process for producing high yields of IFN-$\gamma$ and its mRNA which is reliable, efficient and functional. It also discloses genetic engineering techniques for producing clones which can express IFN-$\gamma$.

Generally, the process involves the production of high yields of IFN-$\gamma$ and its mRNA by incubating a viable cell suspension that comprises leukocytes, an inducer for production of IFN-δ, and a modulator for the inducer. The leukocytes may originate from a variety of sources including human, monkey, sheep, cow, pig and chicken. Particularly, the viable cell suspension includes leukocytes, a modulator selected from the group consisting of mezerein or 12,13-phorbol dibutyrate, and an IFN-δ inducer. Preferably, the IFN-δ inducer is a calcium ionophore selected from the group consisting of A-23187, ionomycin, or 2,2-dimethyl-6,6,7,7,8,8,8-heptafluoro-3,5-octanedione (hereinafter "FOD").

At the end of the incubation period, both IFN-δ and IFN-δ mRNA may be extracted from the cell suspension. The IFN-δ is found in the supernatant and may be purified to homogeneity by adsorbing and eluting the crude IFN-δ through a series of columns. A technique for purifying IFN-δ is disclosed in our copending U.S. application Serial No. 293,775 which is incorporated herein by reference.

The IFN-δ mRNA is contained in the cells. It is extracted from the cells by a process which generally involves first removing the large DNA strands and proteins from the lysed cells, and then separating the mRNA from other types of RNA and small strands of DNA

in a poly (U) column. The mRNA that contains high
IFN-$\gamma$ activity is selected by size fractionation.

This mRNA is now useful for expressing IFN-
in clones. The principle advantage of cloning is that
by placing the DNA which codes for IFN-$\gamma$ into a host
cell, such as bacteria or yeast, there is a potential
for harvesting enormous amounts of IFN-$\gamma$. Briefly,
this is accomplished by adding the isolated mRNA to
a viral enzyme, reverse transcriptase, which reads the
mRNA code and transcribes a single-stranded complementary
DNA. Double-stranded DNA is achieved in the presence
of DNA polymerase or reverse transcriptase, and is placed
into a host cell, such as the bacteria E. coli, by
means of genetic engineering recombinant techniques
such as described in U.S. Patent 4,237,224. For example, insertion of the double-stranded DNA into the
host cell is accomplished by first selecting a cloning
vehicle, such as a plasmid, which is compatible with
the host cell and carries a phenotypical trait, such
as penicillin resistance. Enzymes which are capable
of cleaving specific cites on the cloning vehicle remove a portion of the vehicle approximately the size
of the DNA coding for IFN-$\gamma$ to be inserted. The DNA
and the cloning vehicle are joined together with DNA
ligase. The recombinant cloning vehicle is then introduced into the host cell in the presence of calcium chloride by the process of transformation. Afterwards the cells that have taken up the recombinant cloning vehicle molecule, the transformants, are selected, usually by use of antibiotics, and thereafter selectively isolated by their ability to hybridize with mRNA from IFN-$\delta$ induced cells. These transformants are then screened for expression of IFN-$\delta$. The IFN-$\delta$ ds-DNA from selected transformants are placed in optimal expressing vehicles for large-scale production of IFN-$\delta$.

The calcium ionophores, A-23187 and ionomycin, are antibiotics produced by microorganisms. The calcium ionophore FOD is synthetic material, and it has been shown that its biological effects resemble those described for A-23187. The main difference between FOD and A-23187, for purposes of this invention, is that greater concentrations of FOD appear to be necessary to produce similar yields of IFN-$\delta$. However, there may prove to be no significant distinction in optimal concentrations of A-23187 and FOD upon discovery of a solvent for FOD which is capable of reducing FOD to its monomeric (non-micellar) form and is not cytotoxic. Practically, the use of greater concentrations of FOD is irrelevant since FOD is inexpensive and readily available in large quantities.

Mezerein, the modulator, is a poly-cyclic $C_{38}H_{38}O_{10}$ hydrocarbon with molecular weight of 654. It is an extract from the plant Daphne mezereun L. Mezerein has been shown to be as effective a lymphocyte mitogen as the potent tumor promoting agent, 12-0-tetradecanoylphorbol 13-acetate (TPA). Thus, its discovery as a potent modulator for IFN-δ inducers makes it extremely useful.

The alternative modulator useful in this invention, 12,13-phorbol dibutyrate, is a phorbol ester which, like mezerein, is a potent modulator for IFN-δ inducers.

The combination of the modulator and the inducer produces significantly more titer of IFN-δ than were they used individually, and more than would be expected if the effects of the two were merely additive.

It has also been demonstrated that IFN-δ inducers, particularly one of the calcium ionophores, in combination with a modulator stimulates production of higher immune IFN-δ titer than the unmodulated IFN-δ inducers, staphylococcal enterotoxin A, phytohemagglutinin A and concanavalin A.

The combination of mezerein and calcium ionophore inducers, particularly A-23187, reliably and consistently produces high yields of IFN-δ. Additionally, by virtue

of the relatively low molecular weights of calcium ionophore and mezerein, their hydrophobicity, and in the case of A-23187, its high affinity for certain divalent cations, they are easily removed from the IFN-$\delta$ product. Thus, there is far less concern with toxicity of the mezerein/calcium ionophore system than with the other known inducers which are all proteins and are difficult to remove from other proteins found in the cell suspension. In addition, the inducer proteins may tend to co-purify with the IFN-$\delta$ thereby contaminating the final product, making it impractical for clinical use. Thus, the mezerein/calcium ionophore system is particularly advantageous for production of human IFN-$\delta$ for use in clinical trials, and also for recovery of the mRNA from the incubated cell suspension for future cloning.

It is the general object of this invention to provide processes for producing high yields of IFN-$\delta$. Consequently, the processes provide methods for preparing commercial quantities of crude IFN-$\delta$. It is a further object of this invention to provide IFN-$\delta$ which has been purified to homogeneity, for example, in accordance with the process disclosed in our copending U.S. application Serial No. 293,775.

The objects of this invention also include the provision of processes for producing and isolating IFN-$\delta$ mRNA, which may be used in producing its complementary DNA, and then for expressing IFN-$\delta$ in clones.

According to our invention, we provide a process for the production of IFN-ɣ or its mRNA comprising incubating for a period of time at least sufficient for the production of IFN-ɣ, a viable cell suspension which includes (a) leukocytes, (b) an inducer for the production of IFN-ɣ, and (c) a modulator for said inducer selected from mezerein and 12,13-phorbol butyrate, said inducer and said modulator being present in said suspension in amounts sufficient to stimulate the production of IFN-ɣ.

According to a particular feature of the invention, the inducer is a calcium ionophore selected from A-23187, 2,2-dimethyl-6,6,7,7,8,8,8-heptafluoro-3,5-octanedione (FOD), and ionomycin.

The leukocytes may be obtained from animals of the group comprising human, monkey, sheep, cow, pig and chicken. According to a particular embodiment, the leukocytes comprise human peripheral blood leukocytes.

Prior to the incubation step, the human leukocytes may be suspended in a culture medium, preferably in a proportion of from $1.0 \times 10^6$ to $9.0 \times 10^6$ cells/ml. of cell suspension.

According to a particular feature, the leukocytes are contained in buffy coats. Preferably, prior to the suspending step, erythrocytes are separated from the buffy coats.

Examples of specific culture media are RPMI 1640 and Dulbecco's Minimal Essential Media. The culture medium preferably contains an antibiotic in an amount sufficient to prevent contaminating bacterial growth. Such antibiotic may be a broad spectrum antibiotic selected from 100 units of penicillin per ml. of culture medium, 100 µg. of

streptomycin per ml. culture medium and 100 µg. of gentamicin per ml. culture medium.

In one aspect of the invention, the modulator is mezerein, and is generally included in the suspension in a minimum proportion of 0.7 ng./ml. of cell suspension. The mezerein is preferably included in the suspension in a proportion of 7-30 ng./ml. of cell suspension.

In another aspect of the invention, the modulator is 12,13 phorbol dibutyrate, and is generally included in the suspension in a minimum proportion of 0.7 ng./ml. of cell suspension.

In a preferred embodiment, the 12,13 phorbol dibutyrate is included in the suspension in a proportion of 7-30 ng./ml. of cell suspension. The optimal proportion of modulator to be included in the cell suspension is in the range of the first point of the plateau for the dose response for the modulator.

One example of inducer is calcium ionophore A-23187 which is generally included in the suspension in a proportion of 0.10-10.0 µg./ml. of cell suspension. The calcium ionophore A-23187 is preferably included in the suspension in a proportion of 0.10-2.0 µg./ml. of cell suspension. Another example of inducer is calcium ionophore FOD which is generally included in the suspension in a proportion of 0.10-300 µg./ml. of cell suspension.

A nutrient source may be added to said cell suspension prior to the termination of said incubation. In that event, the nutrient source is generally added to the cell suspension after the cell suspension has been conditioned at approximately body temperature for 2-6 hours. The nutrient source may be, for example, fetal-calf serum or calf serum. The nutrient source is generally added in a proportion of 3%-15% (volume/volume) serum/cell suspension. The nutrient source is generally added in a proportion of 4%-10% (volume/volume) serum/cell suspension.

During the incubation, the cell suspension is generally maintained in a culture container such that there is a dynamic exchange of metabolites between the cell suspension and the air volume above the cell suspension. The air volume above the cell suspension is preferably maintained at a $CO_2$ concentration of 5%. The cell suspension is buffered to a pH such that cells in the suspension remain viable during the incubation. In general, the cell suspension is initially buffered to a pH of 7.2-7.4. The incubation period is normally at least 12 hours, and, preferably, 3-4 days.

In one embodiment of the invention, the cell suspension is incubated under relatively stationary conditions. When the inducer is calcium ionophore A-23187, it is generally included in the suspension in a proportion of 0.15-0.8 µg./ml. of cell suspension, preferably 0.25-0.6 µg./ml. of cell suspension. In another embodiment of the invention, the cell suspension is agitated during incubation. Subsequent to the incubation, the supernatant from said cell suspension, which

contains IFN-ɣ, is generally separated from the cells, for example, by centrifuging. This is generally followed by the step of purifying the IFN-ɣ product.

One specific treatment comprises:

(a) adsorbing said IFN-ɣ onto a column containing Controlled Pore Glass beads and eluting with ammonium sulfate;

(b) adsorbing said IFN-ɣ containing eluate of step (a) onto a column containing Concanavalin A-Sepharose, lentil lectin-Sepharose or pea lectin-agarose and eluting with a buffer containing a sugar;

(c) adsorbing said IFN-ɣ containing eluate of step (b) onto a column containing Heparin-Sepharose or Procian Reg-agarose and eluting with a buffer containing a high salt content; and

(d) passing the IFN-ɣ containing eluate of step (c) through a gel-filtration column equilibrate in high salt and recovering purified IFN-ɣ.

An alternative purification treatment comprises:

(a) (i) contacting said IFN-ɣ with Controlled Pore Glass beads for a period of time sufficient to adsorb said IFN-ɣ onto said beads in the presence of a neutral pH buffer;

(ii) washing said beads with a chemically compatible buffer until the optical density of the eluant at 280 nm is 0;

(iii) washing said beads with a neutral pH buffer solution until the optical density of the eluate at 280 nm is O; and

(iv) eluating IFN-𝛿 from said beads with an ammonium sulfate solution;

(b) (i) contacting said eluate of step (a)(iv) with an adsorbent selected from the group consisting of Concanavalin A-Sepharose, lentil lectin-Sepharose and pea lectin-agarose for a period of time sufficient to adsorb said IFN-𝛿 onto said adsorbent in the presence of a neutral pH buffer;

(ii) washing said adsorbent with ammonium sulfate until the optical density of the eluant at 280 nm is O;

(iii) washing said adsorbent with a neutral pH buffer until the optical density of the eluate at 280 nm is O; and

(iv) eluting IFN-𝛿 from said adsorbent with a buffer containing a sugar of the group consisting of alpha-methyl D-mannoside and 1-methyl D-glucoside;

(c) (i) contacting said eluate of step (b)(iv) with an adsorbent selected from the group consisting of Heparin-Sepharose and Procian Red-agarose for a period of time sufficient to adsorb said IFN-𝛿 onto said adsorbent in the presence of a netural pH buffer;

(ii) washing said adsorbent with a neutral pH buffer containing a sugar selected from the group consisting of alpha-methyl D-mannoside and 1-methyl D-glucoside until the optical density of the eluate at 280 nm is O;

-15-

(iii) washing said adsorbent with a neutral pH buffer until the optical density of the eluate is O; and

(iv) eluting IFN-$\gamma$ from said adsorbent with a neutral pH buffer containing a high concentration of salt; and

(d) (i) passing said eluate of step (c)(iv) through a gel-filtration column equilibrated in a neutral pH buffer containing a high concentration of salt; and

(ii) eluting IFN-$\gamma$ from said column with a neutral pH buffer.

In the last-mentioned treatments, homogeneous IFN-$\gamma$ may be recovered from the eluate of step (d)(ii). The steps of the purification treatment may be carried out continuously or, in the alternative, sequentially. In the last-mentioned alternative purification treatment step (b) may be carried out prior to step (a). In another embodiment, the sequence of steps may be from step (c) to step (b) to step (a) to step (d). In the above alternative purification treatment the IFN-$\gamma$ in said supernatant may be produced from cell suspensions containing unlysed red blood cells, and, prior to beginning said purification steps, the supernatant may have added thereto sufficient tris-(hydroxymethyl)-aminomethane to bring the concentration to 500 mM, and be buffered to pH 9.5 to 9.7. In the alternative purification treatment, the Controlled Pore Glass beads preferably have a mesh size designated as 120/200 or 200/400. Furthermore, in step (a), the chemically compatible buffer is preferably tris-(hydroxymethyl)-aminomethane.

In step (a), the chemically compatible buffer is preferably tris-(hydroxymethyl)-aminomethane at a concentration of 500 mM and pH 9.5, the neutral pH buffer is preferably phosphate buffered saline and the eluant of step (a)(iv) is preferably 2M ammonium sulfate at pH 9.0. In step (b) the neutral pH buffer is preferably phosphate buffered saline, and the buffer containing sugar is preferably phosphate buffered saline containing from 0.1M to 2M alpha-methyl D-mannoside of 1-methyl D-glycoside. In step (c) the neutral pH buffered solution is preferably phosphate buffered saline and the neutral pH buffer of step (c) (iv) is preferably phosphate buffered saline containing 2M sodium chloride. In step (d), the gel-filtration treatment preferably includes equilibrating the column with phosphate buffered saline containing 2M sodium chloride, and eluting with 2M sodium chloride in phosphate buffered saline to achieve separation of the IFN-δ from the solutes. In the final elution, the IFN-δ is preferably further contacted with phenyl-Sepharose for a period of time sufficient to accomplish equilibrated adsorption of said IFN-δ or the phenyl-Sepharose, and the adsorbed IFN-δ is preferably eluted with low salt buffers.

A first specific embodiment of the purification treatment comprises:

(a) contacting said IFN-δ with a resin selected from the group consisting of Heparin-Sepharose and Procian-Red agarose for a period of time sufficient to accomplish equilibrated adsorption of said IFN-δ to the resin;

(b) washing said resin with phosphate buffered saline solution containing a sugar selected from the group consisting of from O.1M to 2M alpha-methyl D-mannoside and 1-methyl D-glucoside until the optical density at 280 nm is O;

(c) washing with phosphate buffered saline solution until the optical density at 280 nm is O;

(d) eluting the adsorbent IFN-ɤ with phosphate buffered saline containing 2M sodium chloride; and

(e) recovering substantially pure IFN-ɤ.

A second specific embodiment of the purification treatment comprises:

(a) contacting said IFN-ɤ with Controlled Pore Glass beads for a period of time sufficient to adsorb said IFN-ɤ on to said beads in the presence of a neutral pH buffer;

(b) washing said beads with a chemically compatible buffer until the optical density of the eluant at 280 nm is about O;

(c) washing said beads with a neutral pH buffered solution until the optical density of the eluant at 280 nm is about O;

(d) eluting IFN-ɤ from said beads with an ammonium sulfate solution; and

(e) recovering substantially pure IFN-ɤ.

In the later embodiment, the chemically compatible buffer is preferably tris-(hydroxymethyl)-aminomethane at a concentration 500 mM at pH 9.5, the neutral pH buffer is preferably phosphate buffered saline, and the final eluant is preferably 2M ammonium sulfate at pH 9.0.

-18-

The production of IFN-$\gamma$ mRNA according to the invention generally includes the step of recovering IFN-$\gamma$ mRNA from the cells subsequent to incubation.

In a particular embodiment, the said leukocytes comprise human leukocytes, and, prior to the incubation, the leukocytes are suspended in a culture medium containing antibiotics. The inducer is preferably a calcium ionophore selected from A-23187, ionomycin and FOD.

In one embodiment, the inducer is A-23187 and is included in the cell suspension in a proportion of 0.10-10.0 µg./ml. of cell suspension. The modulator is preferably mezerein which is included in the suspension in a proportion of 0.7-100 ng./ml. of cell suspension. During the incubation the cell suspension is generally initially buffered to a pH of 7.0-7.6, and the incubation period is generally up to 3 days.

The cells of the cell suspension are generally separated from the supernatant subsequent to incubation.

In one embodiment, IFN-$\gamma$ mRNA is extracted from the separated cells. The extraction process of the IFN-$\gamma$ mRNA may comprise:

(a) lysing the cells and removing large contaminants;

(b) precipitating remaining RNA in alcohol and then resuspending in a salt buffer;

(c) passing the RNA suspension through a poly-(U) column;

(d) eluting the mRNA from said column; and

(e) size fractioning the eluted mRNA.

In a specific embodiment, the extraction comprises:

(a) lysing said cells in a solution comprising sodium acetate and SDS, or guanidine thiocyanate;

(b) removing large strands of DNA and protein by phenol extraction or centrifugation with cesium chloride in a cellulose nitrate tube;

(c) precipitating the remaining mRNA in ethanol and resuspending in a salt buffer;

(d) passing the mRNA suspension through a poly-(U) Sepharose-4B column and washing with a buffer;

(e) eluting the mRNA from said column with formamide; and

(f) size fractioning the eluted mRNA in sucrose gradient.

Preferably, samples of the fractions of extracted IFN-ɤ mRNA are inserted into cells capable of expressing IFN-ɤ, the cells are allowed to express the IFN-ɤ, and the quantitative expression of IFN-ɤ by the cells is measured.

According to a particular aspect of our invention, we provide a process for producing clones that express IFN-ɤ comprising:

(a) adding reverse transcriptase to IFN-ɤ mRNA from high IFN-ɤ activity fractions of claim 35, to transcribe the single-stranded complementary IFN-ɤ DNA;

(b) adding DNA polymerase or reverse transcriptase to said single-strand DNA to produce double-stranded IFN-ɤ DNA;

(c) making the IFN-ɣ ds-DNA blunt-ended by incubation with nuclease;

(d) selecting a cloning vehicle carrying a pheno typical trait, said vehicle being compatible with a host cell;

(e) cleaving said cloning vehicle in the presence of restrictive endonuclease;

(f) combining said cleaved vehicle with said IFN-ɣ double-stranded DNA in the presence of DNA ligase to form a recombinant vehicle;

(g) adding complementary terminal codons to the blunt-ended IFN-ɣ ds-DNA and the cleaved cloning vehicle in the presence of terminal transferase;

(h) transforming the host cell with the recombinant vehicle in the presence of calcium chloride;

(i) growing said host cells and then isolating the transformant containing the recombinant vehicles by means of the phenotypical trait.

According to another aspect of the invention, a process for expressing IFN-ɣ comprises:

(a) selecting the transformants of step (i) of claim 36 which demonstrate strong hybridization to mRNA from IFN-ɣ induced cells; and

(b) removing IFN-ɣ ds-DNA from said transformants and inserting it in an optimal expression cloning vehicle.

Particular products which are characteristic of the present invention comprise:

(A) Homogeneous IFN-ɣ;

(B) Homogeneous human IFN-ɣ;

(C) The IFN-ɣ$_I$ species of said homogeneous human IFN-ɣ corresponding to SDS-PAGE molecular weight band of 25,000 ± 3,000 daltons;

(D) The dimer of said IFN-$\alpha_1$ species corresponding to an SDS-PAGE molecular weight band of 47,000 ± 3,000 daltons;

(E) The trimer of said IFN-$\alpha_1$ species corresponding to an SDS-PAGE molecular weight band of 63,000 ± 3,000 daltons;

(F) The IFN-$\alpha_2$ species of said homogeneous human IFN-$\alpha$ corresponding to an SDS-PAGE molecular weight band of 20,000 ± 3,000 daltons;

(G) The recombinant cloning vehicle containing DNA segment coding for human IFN-$\alpha$; and

(H) The host cell containing the recombinant cloning vehicle just mentioned.

Other objects, features and advantages will be evident from the following description of the invention.

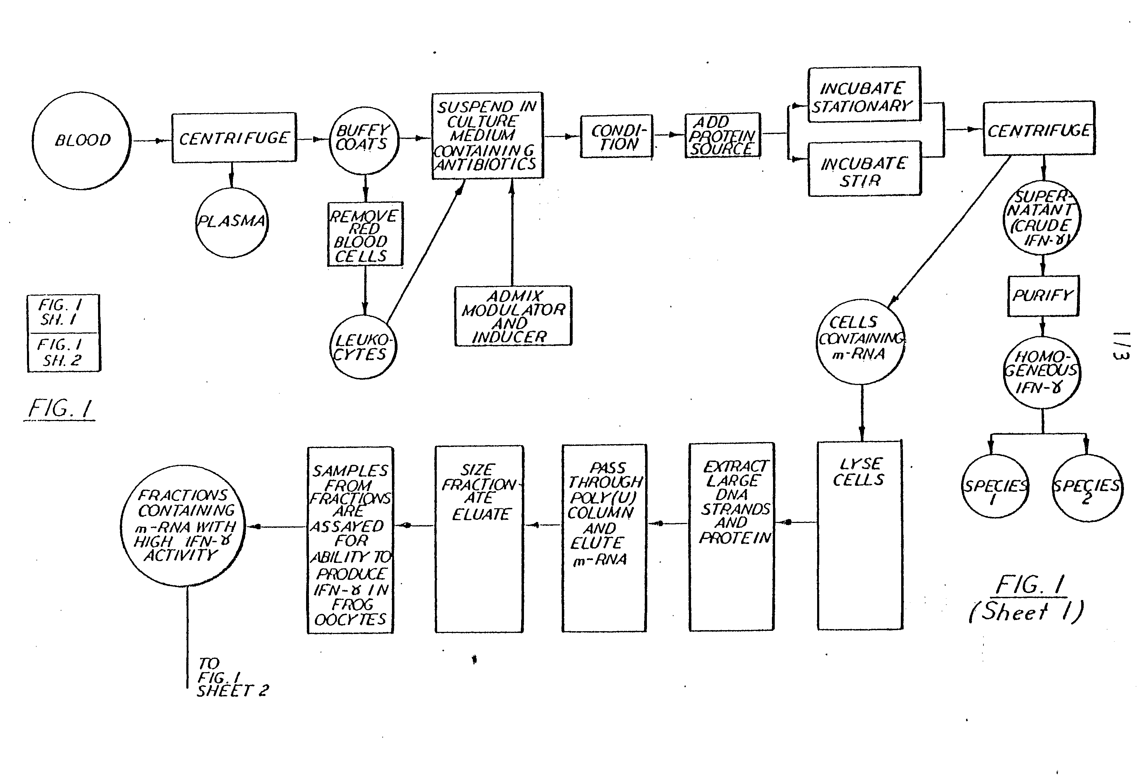

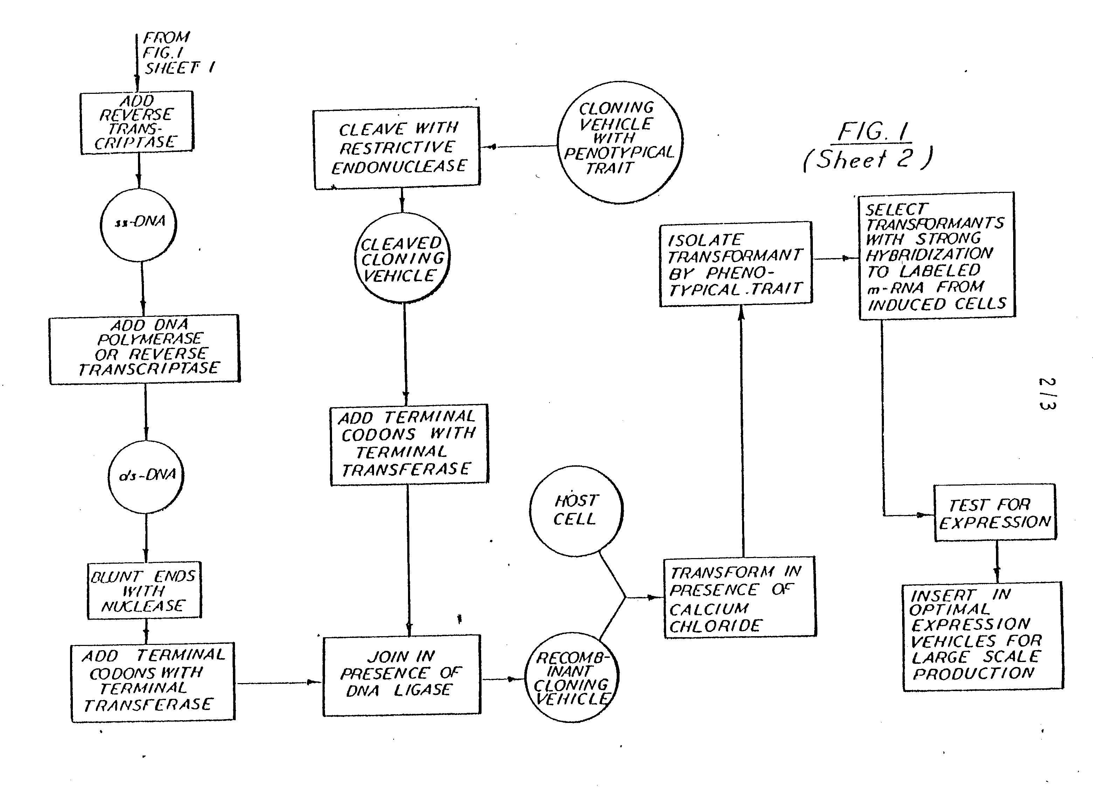

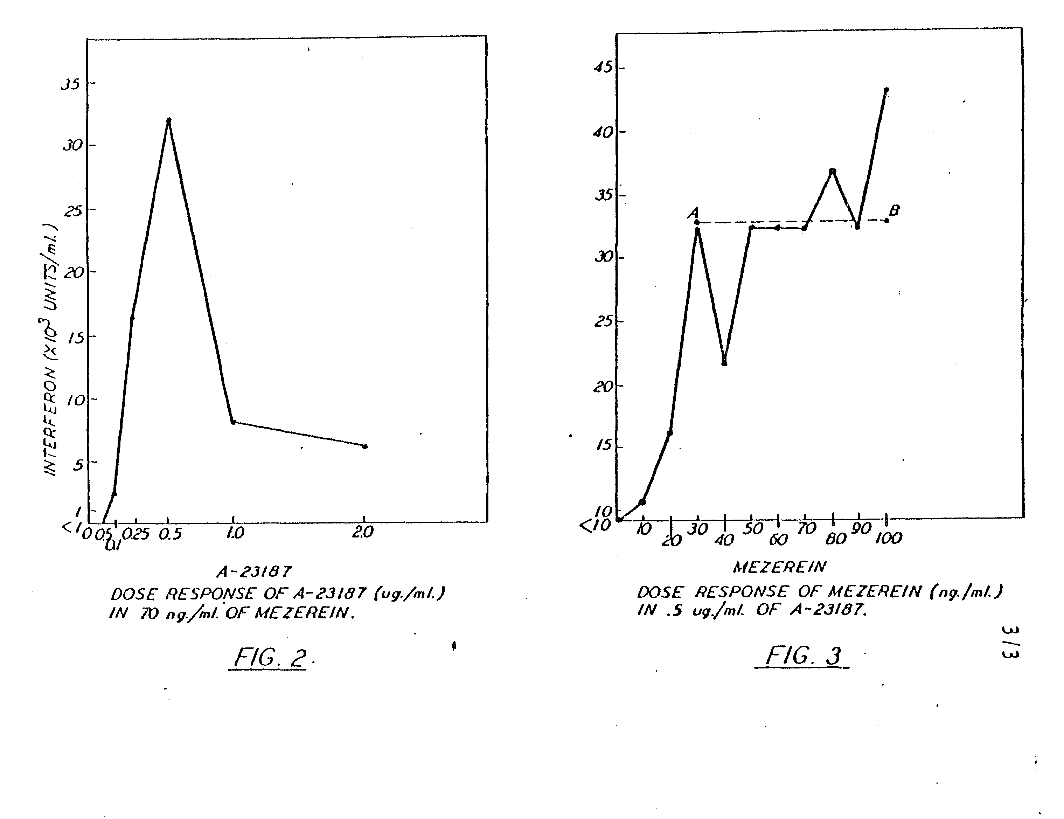

The accompanying drawings will assist in understanding the invention disclosed herein. Figure 1 is a generalized flow chart of the various process steps. Figures 2 and 3 are dose response curves associated with Example 4 herein. The Figure 1 flow chart is a diagrammatic aid for the specification and claims and does not fully disclose all the features of this invention which are described in this specification.

The flow chart describes the process for production of IFN-$\delta$ and its purification to homogeneity; the process for production of mRNA useful for cloning IFN-$\delta$; and the process for producing clones capable of expressing IFN-$\delta$.

With reference to the Figure 1 flow chart, buffy coats are isolated from whole blood by centrifugation and then suspended in a culture medium containing antibiotics. Alternatively, the erythrocytes are removed from the buffy coats and the remaining leukocytes are suspended in the culture medium. The modulator and inducer are admixed with the suspension and
conditioned for a short period. A nutrient source is
added and the cell suspension is incubated under either
stationary or agitated conditions. The incubated cell
suspension is then centrifuged to separate the supernatant from the cells. The supernatant contains the
crude IFN-δ which is purified to homogeneity. The
cells contain the IFN-δ mRNA which is used in the cloning process.

The cells are lysed and the IFN-δ mRNA is
extracted by a process comprising removing the large
DNA strands and proteins, passing the remaining RNA
through a poly (U) column to remove rRNA and small
strands of DNA, eluting mRNA from the column and
size fractionating the mRNA. Samples from the various
sizing fractions are assayed for their ability to
produce IFN-δ in frog oocytes. The fractions corresponding to the highest IFN-δ activity are selected for
cloning.

The ds-DNA to be inserted in the cloning
vehicle is prepared from the extracted mRNA in two stages.
First the ss-DNA complementary to the mRNA is synthesized
in the presence of reverse transcriptase. This ss-DNA acts as the template to synthesize its complementary ss-DNA in the presence of reverse transcriptase or DNA polymerase, and through hybridization achieving the ds-DNA. The ds-DNA is made blunt ended and appropriate terminal codons are added. The ds-DNA is then joined with a cleaved cloning vehicle with appropriate terminal condons in the presence of DNA ligase. This recombinant cloning vehicle is placed in the host cell by the transformation process. The resulting transformants are isolated by means of the phenotypical trait carried by the cloning vehicle. The transformants are further selected by their ability for strong hybridization to labelled mRNA from IFN-induced cells. The selected transformants are tested for ability to express IFN-𝛿 . These transformants which express IFN-𝛿 may be grown to large numbers in fermenters or preferably the IFN-𝛿 ds-DNA from these transformants may be removed and inserted into optimal expressing vehicles which may be grown for large-scale production of IFN-𝛿 .

In accordance with the preferred embodiments of this invention, buffy coats containing human leukocytes are provided as a viable cell source for human IFN-𝛿 production. Buffy coats may be obtained by a variety of standard clinical techniques, such as by leukophoresis or on a hemonetics machine.

One suitable method involves collecting whole blood (usually 100 ml.) by venipuncture from healthy donors in acid-citrate-dextrose solution or in heparinized containers. The acid-citrate-dextrose and heparin are normal additives to blood units preventing coagulation of the blood cells upon standing. The whole blood is centrifuged and the plasma fraction removed. The layer immediately beneath the serum of the centrifuged material is the buffy coat. The buffy coat consists primarily of leukocytes and some erythrocytes. One component of the leukocytes are T-cell lymphocytes. It is believed that these lymphocytes are responsible for production of IFN-$\gamma$ .

Another method of isolating the leukocytes is by the Ficoll-Hypaque gradient method as described in A. Boyum, "Isolation of Leukocytes from Human Blood", 21 Scand. J. Clin. Lab. Invest. Suppln. 31-50 (1968). A substantial portion of the cells derived from this technique are lymphocytes, of which the T-cell originated lymphocytes are present. The remaining mononuclear leukocyte cells are B-cell lymphocytes, monocytes and null cells.

In copending application Serial No. 255,138 a process was disclosed in which human peripheral blood was treated with a buffered ammonium chloride solution to lyse the erythrocytes, followed by centrifuging to remove the lysed material. It has now been discovered that, when the preferred inducers are used, yields of human IFN-$\delta$ are improved up to five times if the buffy coat material is not pretreated with the ammonium chloride solution. In the preferred embodiment, buffy coat material is used directly in the modulation-induction procedure of this invention.

The buffy coat material is suspended in a culture medium, with from $1.0 \times 10^6$ to 9.0 to $10^6$ leukocyte cells/ml. of cell suspension as the concentration range. The optimum concentration range is from $4.0 \times 10^6$ to $7.0 \times 10^6$ cells/ml. The preferred concentration is $5.0 \times 10^6$ cells/ml. Commercially available culture media which have been found to be especially suitable are RPMI 1640 available from GIBCO Laboratories, Grand Island, N.Y., and when using the preferred inducers, Dulbecco's Minimal Essential Media (DMEM) available from Meloy Laboratories, Inc., Springfield, Virginia.

In some cases, it is desirable to add antibiotics to the culture medium before suspending the buffy coats to prevent contaminating bacterial growth. Broad spectrum antibiotics such as 100 units of pennicilli per ml. culture medium, 100 μg. of strepto-
mycin per ml. culture medium, or 100 μg. of gentamicin
per ml. culture medium may be used. Although the
inclusion of antibiotics in the culture medium inhibits
bacterial growth, the antibiotics themselves are
contaminants in the sense that they must be removed
from the extracted interferon product. Even trace
amounts of antibiotics, if not completely removed
from the interferon product, may cause allergic
reactions when administered to susceptible patients.

A modulator selected from the group consisting
of mezerein and 12,13-phorbol dibutyrate is added to
the culture medium containing the suspended buffy coat
material. These modulators are commercially available
from CCR, Inc., Eden Prairie, Minnesota. The modulator
may be added in a minimum amount of        0.7 ng./ml.
of cell suspension. The upper limit does not appear
to be critical because at optimal concentrations
of modulator the yield of IFN-$\gamma$ plateaus. There may
be a divergence in the optimal concentration of
modulator among different preparations or batches;
this is likely due to the level of purity of the
modulator in each batch. The purer the modulator,
the less that is necessary to produce maximum yields
of IFN-$\gamma$. The optimal amount of modulator to be used for a particular batch of modulator is preferably determined by constructing a calibration grid. (See, e.g., Example 4, and Figure 3.) This may be done by plotting a series of dose responses for varying concentrations of mezerein as a function of varying concentrations of the inducer. The optimal proportion of modulator to be included in the cell suspension is in the range of the first point of the plateau of the dose response curve (e.g., dotted line segment A-B of Figure 3). Based on the calibration grids constructed for two separate batches of mezerein, the minimum concentration for mezerein to induce optimal quantities of IFN-$\gamma$ varies between 7 ng./ml. and 30 ng./ml. Although it has been found that the concentration of mezerein required for optimal modulation of A-23187 may vary from batch to batch, neither the kinetics of IFN-$\gamma$ production nor the shape of the A-23187 dose response curve is altered.

Serial No. 255,138 described introduction of calcium ionophore A-23187 and a modulator into the culture medium that contains peripheral blood leukocytes. The amount of A-23187 per ml. of cell suspension was in the range of 0.1-10.0 µg./ml., and preferably 0.10-2.0 µg./ml. Thereafter, the mixture was incubated for between 12 hours and 7 days at 37°C. while gently stirring the culture (spin culture) at a rate of    10 rpm. The spin culture suspension was maintained under a blanket of air/$CO_2$, which can be maintained by feeding $CO_2$ into the suspension through micron pore size air stones to distribute the gas. Thus, a uniform $CO_2$ mixture of 5% was maintained at all times.

It has now been found that A-23187 may very effectively be used within the preferred proportional range of 0.15-0.8 ug./ml. of cell suspension under stationary culture conditions during the incubation period. Preferably, A-23187 in the amount of between 0.25-0.6 ug./ml. is used. Within this range, stationary cultures have yielded 2-3 times higher titers of IFN- than when the cultures are agitated.

The incubation temperature should remain at  body temperature, i.e., 37°C. The temperature may be varied to the extent the cells remain viable, and within the range of viability, there appears to be a relationship between temperature and incubation time: higher temperatures appear to require shorter incubation times. Induction of IFN- $\delta$ has been observed after    12 hours of incubation, and induction lasts until the cells are no longer viable, up to as long as    12 days. Three to 4 days is the preferred incubation period on the basis of maximal yield per unit of time.

It has been demonstrated that there is an initial phase of production between 20 hours and 40 hours. This is followed by a loss of activity and then a second phase of production.

The greatly improved yield of IFN-$\gamma$ resulting from the stationary culture incubation and the smaller preferred proportion of A-23187 is thought to be due to the availability of direct cell contact of adjacent cells throughout the stationary incubation period, rather than continual random cell contact which occurs when the cell suspension is agitated during the incubation period. In any event, increased yields of up to 3 times have been noted.

In addition to increased yields, the stationary culture incubation process also permits considerably smaller batches to be made that yield the same proportion of IFN-$\gamma$ as when the larger batch is used in the spinner culture incubation process. As a consequence, there is a significant reduction in the amount of contaminants that co-reside with the IFN-$\gamma$. Moreover, there is a significant cost saving by using less A-23187. Thus, without reducing the overall yield, the efficiency of the earlier process is materially improved.

A-23187 alone will induce cells to produce barely detectable amounts of human IFN-$\gamma$. By a mechanism which is not entirely understood, the addition of a modulator appears to increase the number of cells capable of responding to A-23187, and thus greatly enhances the yield of IFN-$\gamma$. For instance, in reproduction of the Dianzani et al. article (calcium ionophore A-23187, was employed without a modulator), only 10-20 units of antiviral activity per ml. was measured. In contrast, the combination of mezerein with an inducer has consistently induced cells to increased yields of IFN-$\gamma$. Particularly, mezerein in combination with A-23187 produces IFN-$\gamma$ in the range of up to 400,000 and more units of anti-viral activity per ml.

An alternative calcium ionophore which is a practical and effective inducer is 2,2-dimethyl-6,6,7,7,8,8,8-heptafluro-3,5-octanedione (FOD). It is commercially available from the Aldrich Chemical Company, catalog #17,516-L. FOD has been demonstrated to closely resemble calcium ionophore A-23187 in its effects on biological systems. ("A Synthetic Ionophore For $Ca^{2+}$: Studies With Model And Biological Systems", Gomperts, et al., 117 Eur. J. Biochem., pp. 559-562, 1981).

Ionomycin is also a potent IFN-$\gamma$ inducer. It

is a calcium ionophore available from Squibb, Inc.

Experiments using FOD as an inducer for IFN-δ production ranging from 30-300 μg./ml. of cell suspension have been conducted. While FOD has been added at concentrations 100-1,000 times greater than A-23187 it is believed that the greater FOD concentration requirement may be due to its being in a micellar state. Use of a solvent for FOD which is capable of reducing it to its monomeric (non-micellar) form and which is not cytotoxic will eliminate this variance in concentrations between the two calcium ionophores. In the presence of such a solvent the minimum concentration proportion for FOD will be as low as .10 μg./ml. of cell suspension. As a practical matter the high concentrations of FOD are not significant because it is inexpensive and readily available in large quantities.

It should be noted that the order in which the modulator and calcium ionophore inducer are included in the cell suspension is not controlling. They may be added in any order, or simultaneously. However, when the inducer is a protein it should be added approximately 2 hours after the modulator.

It has also been found that IFN-δ yield is further increased if a nutrient source such as a protein source, for example, fetal calf serum or calf serum is added to the cell suspension in an amount sufficient to keep the cells alive. That amount is generally 3% to 15% at final concentration and, preferably, from 4% to 10% (volume/volume) serum/cell suspension. It is preferable to add the nutrient source after the cell suspension has been conditioned for 2-6 hours at body temperature.

It is essential to the invention that the cells remain viable during incubation. Thus, the appropriate amount of cell suspension to be included in a culture container is determined by the capacity of that particular container such that a suitable volume of air/$CO_2$ makes contact with the cell suspension to maintain viability over the incubation period. Conditions must be such that a dynamic exchange of $CO_2$ and other volatile metabolites of the cell suspension can be established between the suspension media and the air volume. Suitably, relatively shallow apparatus, with liquid depths such as approximately 3mm. in deep trays or 5-10mm. in tissue culture tubes are used to carry the stationary culture during incubation, thereby providing a large surface area-to-volume ratio.

It is desirable to initiate the incubation period with a pH such that the cells will remain viable.

For example, the culture may be initially buffered to a pH of 7.0-7.6, preferably 7.2.-7.4.

After the cell culture suspension has incubated for a period of time sufficient to produce desired yields of crude IFN-$\gamma$, the cells are separated from the supernatant, for example, by centrifuging, at 1000-3000 x g, depending on the culture volume, for the necessary time, e.g., 10-60 minutes, at 4°C. The yields of crude IFN-$\gamma$ that result from the crude cell suspensions may contain up to 400,000 units and more IFN-$\gamma$ activity per ml. cell suspension. The residual cells can be used for reinducing IFN-$\gamma$ production, or for recovery of IFN-$\gamma$ mRNA, or both. The supernatant containing the crude IFN-$\gamma$ may be stored at 4°C.

A partial purification process for crude IFN-$\gamma$ is described in, "Partial Purification and Characterization of Human (immune) Interferon," Yip et al., 78 <u>Proc. Natl. Acad. Sci. U.S.A.</u>, No. 3, 1601-05, (1981).

A technique for purifying IFN-$\gamma$ is disclosed in Serial No. 293,775. Generally, the process concerns passing the crude IFN-$\gamma$ through 4 columns. One column contains Controlled Pore Glass beads. The crude IFN-$\gamma$ is adsorbed onto the column and eluted with ammonium sulfate. A second column contains either Concanavali-A-Sepharose, lentil lectin-Sepharose or pea lectin-agarose. The IFN-δ is adsorbed onto this column and then eluted with a buffer containing sugar. A third column contains Heparin-Sepharose or Procian Red-agarose. The IFN-δ is adsorbed onto this column and eluted with a salt buffer. The final column is a gel-filtration column equilibrated in high salt.

More specifically; the crude IFN-δ is contacted with Controlled Pore Glass beads in a chromatographic column for a period of time sufficient to acomplish equilibrated adsorption of the crude IFN-δ to the beads in the presence of a neutral pH buffer such as phosphate buffered saline (PBS) at pH 7.2. If the crude IFN-δ is produced from cell suspensions containing unlysed red blood cells, then, before contacting the crude IFN-δ to the CPG beads tris-(hydroxylmethyl)-aminomethane is added to bring the concentration to 500mM and buffered to a pH of 9.5 to 9.7. The glass beads are then washed with a chemically compatible buffer such as an aqueous solution containing tris-(hydroxymethyl)-aminomethane at a concentration of 500mM, and buffered to pH 9.5, to remove the unbound contaminant until the optical density of the eluate at 280 nm. is 0. The glass beads having IFN-δ adsorbed

thereon are next washed with a neutral pH buffer solution until the optical density at 280 nm. is 0, and then the adsorbed IFN-δ is eluted from the glass beads with an aqueous solution of 2M ammonium sulfate at pH        9.0.

This eluate is then passed through a column packed with an adsorbent selected from the group consisting of Concanavalin A-Sepharose, lentil lectin-Sepharose and pea lectin-agarose for  a  period of time sufficient to accomplish equilibrated adsorption of the IFN-δ to the adsorbent in the presence of a neutral pH buffer such as phosphate buffered saline.  The adsorbent having IFN-δ adsorbed thereon is washed with ammonium sulfate until the optical density of the eluate at 280 nm. is        0.  Elution of the adsorbed IFN-δ is then carried out with a phosphate buffered saline containing from 0.1 M to 2.0M alpha-methyl D-mannoside or l-methyl D-glucoside.

The IFN-δ eluate is then contacted with Heparin-Sepharose or Procian Red-agarose and equilibrated in a neutral pH buffered solution.  The IFN-δ is washed with a neutral pH buffered solution such as PBS containing 1 M alpha-methyl D-mannoside or 1-methyl D-glucoside to remove the unbound material until the optical density of the eluate at 280 nm. is        0.  Again, the adsorbent is washed with a neutral pH buffered solution until the optical density is        0 to remove some of the bound material.  The adsorbed IFN-δ is eluted with a second neutral pH buffered solution containing a high concentration of salt.

The IFN-ɣ may be adsorbed and eluted in these three columns in any order.

The IFN-ɣ may be further purified by subjecting the elution fraction to gel-filtration treatment equilibrated in a neutral pH buffered solution containing a high concentration of salt, and then eluting with the same equilibrating buffer.  The solutes within the elution fractions may be separated according to their molecular weights.

Analysis of the resulting eluate material from the gel-filtration column that is passed through SDS-PAGE demonstrated 4 bands.  These are at approximately 20,000 daltons, 25,000 daltons, 47,000 daltons, 63,000 daltons.  The molecular weight measurements are subject to an accuracy of        ± 3,000 daltons.  An Amino acid analysis on each band indicated that the 25,000-63,000 dalton bands have essentially the same amino acid contents, and it is thereupon concluded that the 47,000 and 63,000 bands represent multiples, i.e., dimers and trimers of the 25,000 dalton band. The 25,000-63,000 dalton bands represent the homogeneo species IFN-$\delta_1$. Biological activity assays indicated that the 25,000 and 47,000 dalton bands possess IFN-$\delta$ activity; the 63,000 dalton band IFN-$\delta$ will possess biological activity, but this has not yet been confirm

The 20,000 dalton band represents the homogeneous species IFN-$\delta_2$ since its amino acid analysis is different from that of the 25,000-63,000 dalton ban The 20,000 dalton band IFN-$\delta$ will possess biological activity, but this has not yet been confirmed.

The homogeneous IFN-$\delta$ is particularly useful for physiochemical characterization structure studies, antibody production, and clinical application.

After incubating the cell suspension for up to 3 days, the mRNA may be extracted f.om the cells It is believed that, after 3 days, enzymes start digesting the mRNA. First, the cells are lysed and the large contaminants removed. For example, the cells are isolated by centrifuging and then lysed with sodium acet and SDS or in a guanidine thiocyanate solution. Large strands of DNA and proteins are removed with phenol extraction or by centrifuging in cesium chloride in a cellulose nitrate tube. The remaining mRNA pellet is precipitated in ethanol and then resuspended in a salt buffer. To further remove DNA and rRNA the suspension is bound to a column containing poly (U) sepharose and washed with a buffer. The mRNA is eluted with formamide and size fractionated by sucrose gradients. Samples from the fractions are inserted in cells capable of expressing IFN-$\delta$, such as Xenopus Laevis oocytes. After the oocytes have had sufficient time to express the IFN-$\delta$, the IFN-$\delta$ activity is measured. The fractions corresponding to the highest IFN-$\delta$ are selected for cloning.

The fractions containing high IFN-$\delta$ activity are then added to reverse transcriptase. This enzyme reads the mRNA code and transcribes its single-strand complementary DNA. Reverse transcriptase or DNA polymerase is added to achieve the double-stranded DNA.

This double-stranded DNA may be placed in a host cell, such as the bacterium E. coli, by relying on general genetic-engineering recombinant techniques such as described in the summary.

The preferred embodiments of this invention are further illustrated by the examples which follow.

-40-                                    0063482

## EXAMPLE 1

Production of Crude IFN-$\delta$; Removed Erythrocytes;
Spin Culture

The red blood cells contained in buffy coats were lysed with 7 volumes of 0.83% (weight/volume) buffered ammonium chloride and immediately centrifuged at approximately 25 x g for 20 minutes to separate the lysed red blood cells from the buffy coat containing the leukocytes. The leukocytes were suspended at $5.0 \times 10^6$ cells per mL. in DMEM medium (Meloy).

Mezerein, the modulator, at a final concentration of 7 ng./mL. of cell suspension and the calcium ionophore A-23187, at a final concentration of 2.0 $\mu$g./ml. of cell suspension were admixed with the leukocytes and medium to form the cell suspension. The cell suspension was conditioned for 2 hours at 37°C. The fetal calf serum was then added until the fetal calf serum in the culture reached 10%.

The culture was then incubated for 72 hours at 37°C. in a gently stirred container. After incubation, the culture (crude IFN-$\delta$) was harvested by centrifuging at 2620 x g at 4°C. for 20 minutes. The supernatant containing crude IFN-$\delta$ was separated from the cells and particulate material.

## EXAMPLE 2

### Dose Response for A-23187

IFN-$\delta$ production from human leukocytes as a function of varying A-23187 concentrations modulated by mezerein at a concentration of 7 ng./ml. is shown in the dose response data shown in Table I. The mezerein was prepared from a single batch received from CCR, Inc. The IFN-δ was prepared under stationary incubation conditions (see Example 4).

TABLE I

| A-23187 Added (µg./ml.) | IFN- Titer (Units/ml.) |
|---|---|
| 0.01 | < 30 |
| 0.05 | < 30 |
| 0.10 | < 30 |
| 0.15 | 1200 |
| 0.20 | 360 |
| 0.25 | 720 |
| 0.50 | 720 |
| 1.0 | 120 |
| 10.0 | < 30 |

The optimum yield of IFN-δ was from 0.15 to 1.0 µg./ml. of A-23187 for this particular batch of mezerein.

Table I titers of IFN-δ were measured in a semi-micro c.p.e.-inhibition assay using WISH cells as the indicator cells and Vesicular Stomatitis Virus (VSV) as the challenge virus. Significantly higher titers of IFN-δ were measured when the challenge virus encephalomyocarditis virus (EMC) was used. (See Example

5.) EMC is more sensitive to the anti-viral effects of IFN-$\gamma$.

## EXAMPLE 3

### Modulated A-23187 Verses Unmodulated Protein Inducers

The human IFN-$\gamma$ modulated by mezerein and induced by A-23187, as in Example 1, was compared to IFN-$\gamma$ production induced by optimum concentration of staphylococcal enterotoxin A (SEA), phytohemagglutinin-P (PHA-P) and concanavalin A (Con A), without a modulator. Leukocyte cultures (5 x $10^6$ cells/ml. DMEM medium) were induced with optimal doses of A-23187 (and mezerein), SEA, PHA-P and Con A. Modulated A-23187 induced 2-4, 5-10 and 10-20 times, respectively, more IFN-$\gamma$ than the unmodulated proteins.

Virtually all of the detectable interferon of Examples 1,2 and 3 were IFN-$\gamma$. Treatment at pH 2 resulted in 99% loss of activity in 1 hour, whereas control human IFN-$\alpha$ was only slightly affected. Antibodies to IFN-$\alpha$ had little effect on IFN-$\gamma$, whereas such antibodies completely neutralized the antiviral effect of IFN-$\alpha$. Furthermore, the IFN-$\gamma$ was not active on bovine cells, and it demonstrated a different chromatographic profile when compared to human IFN-$\alpha$.

## EXAMPLE 4

### Production of Crude IFN-$\gamma$ ; Buffy Coats; Stationary Culture

Whole blood, approximately 100 ml. volumes, was collected by venipuncture from healthy human donors in acid-citrate-dextrose solution. The whole blood was centrifuged and the plasma fraction removed. The buffy coat material was suspended at 5.0 x 10$^6$ leukocyte cells per ml. in DMEM medium (Meloy).

Stock solutions of A-23187 and mezerein were prepared in DMSO at concentrations of 50 mg./ml. and 1 mg./ml., respectively. Working solutions were then prepared in DMEM. Mezerein, at a final concentration of 70 ng./ml. of culture, and A-23187, at a final concentration of 0.5 µg./ml., were added to the leukocyte and medium to form the cell suspension. The mezerein used in this example was from a different batch than the mezerein used in Examples 1, 2 and 3. (Upon constructing calibration grids using mezerein in 10 ng./ml. increments ranging from 10 ng./ml. to 100 ng./ml. and A-23187 in varying concentrations ranging from .05 µg./ml. to 2 µg./ml., it was determined that 30 ng./ml. of cell suspension was the optimal concentration of mezerein for this batch. 30 ng./ml. corresponds to the first point of the plateau (e.g., dotted-line segment A-B of Figure 3) of the dose response curve. 70 ng./ml. was used merely as a safety margin.

A dose response curve of A-23187 in 70 ng./ml. of mezerein is shown in Figure 2.

A dose response curve for mezerein in .5 $\mu$g./ml. of A-23187 is shown in Figure 3. The plateau is determined by the average of the dose response points beginning with the point corresponding to the first significant production of IFN-$\gamma$. In this case that point was approximately $32 \times 10^3$ times of IFN-$\gamma$ activity.

The cultures were then poured into tissue culture tubes in which the depth of the culture was approximately 10 mm. and were then conditioned for 2 hours at 37°C. Fetal calf serum was then added until the fetal calf serum in the cell suspension reached 10%. The cell suspension was then incubated for 3 days at 37°C. under stationary conditions and under an air/$CO_2$ blanket containing 5% $CO_2$. At the end of the incubation period, the cultures (crude IFN-$\gamma$) were harvested by centrifuging at 1000 x g at 4°C. .for 60 minutes. The supernatant containing crude IFN- was separated from the cells and particulate material.

EXAMPLE 5

Yield of Crude IFN-$\gamma$

A sample of the IFN-$\gamma$ of Example 4 was titrated in a semi-micro c.p.e.-inhibition assay using WISH cells as the indicator cells and encephalomyocarditis virus (EMC) as the challenge virus. IFN-$\gamma$ was approximately

30 x $10^3$-40 x $10^3$ units/ml.

Virtually all of the detectable interferon in this system was IFN-$\gamma$. Treatment at pH 2 resulted in 99% loss of activity in 1 hour, whereas control human IFN-$\alpha$ was only slightly affected. Antibodies to IFN-$\alpha$ had little effect on IFN-$\gamma$, whereas such antibodies completely neutralized the antiviral effect of IFN-$\alpha$. Furthermore, the IFN-$\gamma$ was not active on bovine cells, and it demonstrated a different chromatographic profile when compared to human IFN-$\alpha$.

Using the method of Example 4 and EMC as the challenge virus, IFN-$\gamma$ titers have ranged from 8000 units/ml. to　　　　400,000 units/ml. and more.

## EXAMPLE 6

### Purification to Homogeneity

The following solutions were prepared: solution of 500 mM tris (hydroxymethyl) amino methane at pH 9.6, then add 12N HCL to bring pH to 9.5, referred to as tris; solution of 2M ammonium sulfate at pH 9.0 (w/NH$_4$OH); neutral pH buffered solution of phosphate buffered saline (PBS) at pH 7.2 having an 1M solution of alpha-methyl D-mannoside; and phosphate buffered saline solution (PBS) at pH 7.2 containing 2M sodium chloride.

The following columns were prepared: 2.6 cm. x 30 cm. chromatographic column was loaded with Con-

trolled Pore Glass beads (CPG) (160 ml. bed volume); 1.6 cm. x 16 cm. chromatographic column was loaded with Concanavalin A-Sepharose (Con A) (32 ml. bed volume); and, an 1.6 cm. x 8 cm. chromatographic column was loaded with Heparin-Sepharose (H/S) ( 16 ml. bed volume). These three columns were equilibrated with phosphate buffered saline (PBS). Two gel-filtration columns, 2.6 cm. x 94 cm., containing P-100 ( 400 ml. bed volume each) were prepared and equilibrated with 2M NaCl in PBS and hooked up in series.

The purification was carried out as follows. A 3.6 liter solution of the centrifuged crude IFN-$\delta$ was adjusted to 500 mM with powdered tris. The adjusted crude IFN-$\delta$ was loaded onto the CPG column at flow rate of 500 ml./hr. (about 94 ml./cm.$^2$/hr.). The column was monitored with an UV monitor at 2.0 AU and the unbound material collected into a beaker. The CPG column was washed with a 500 mM tris solution, pH 9.5, until the optical density ($OD_{280}$) was 0. The column was monitored at 2.0 AU and the unbound material collected. The column was washed with PBS until $OD_{280}$ returned to the baseline. (Flow rate 80 ml./hr. overnight). The eluted material was collected into a second beaker. The CPG column was eluted with 2M ammonium sulfate at pH 9.0 at a flow rate of 24 ml./hr., and 10 ml. fractions were collected. When the second peak began to appear on the monitor, the inlet of the Con A column was connected into the outlet of the monitor and the outlet of the Con A column into the fraction collector. The CPG column eluted onto the Con A column at a flow rate of 24 ml./hr. (12 ml./cm.$^2$/hr. through Con A). The column was monitored as above until the $CD_{280}$ reached the baseline.

The CPG column was disconnected from the monitor and the monitor connected to the Con A column. The Con A column was washed with PBS until $OD_{280}$ returned to the baseline. The flow rates and collections were monitored as above. The inlet of the H/S column was connected to the outlet of the monitor and the outlet of the H/S to the fraction collector. The Con A column was eluted with the PBS sugar solution. The flow rate and collect were monitored as above until the $OD_{280}$ returned to the baseline.

The Con A column was disconnected from the monitor and the H/S connected to the monitor. The H/S column was washed with PBS until $OD_{280}$ returned to the baseline. The flow rate and collection were monitored as above. The H/S column was eluted with 2M NaCl in PBS, pH 7.2 until $OD_{280}$ returned to the baseline. The flow rate was monitored as above and 3 ml. fractions were collected. The H/S peak fractions were selected by $OD_{280}$, and then collected and pooled.

The pooled material was loaded onto the drained bed of the first P-100 column at 20 ml./hr. by gravity ( 80% bed height pressure head). After the sample was loaded, the sides of column were washed with 2M NaCl in PBS, pH 7.2. The flow was stopped and the column topped off with PBS. The flow was then opened and the column was run at 24 ml./hr. in PBS, pH 7.2 with 2M NaCl. The system was monitored at 0.5 AU and 10 ml. fractions were collected.

## EXAMPLE 7

### Yield of Pure IFN-$\gamma$

The results in Table II show the degree of purification and recovery of human IFN-$\gamma$ when purified by the sequential purification techniques described above.

### TABLE II

| Description | Titer ($\log_{10}$ units/ml.) | Volume (ml.) | Recovery (%) | Protein (mg./ml.) | Specific Activity ($\log_{10}$ units/m.) |
|---|---|---|---|---|---|
| Crude | 3.7 | 3600 | NA | 18.46 | 2.4 |
| P-100 Column: | | | | | |
| Fraction 123 | 6.2 | 9.5 | 84 | 0.020 | 7.9 |
| Fraction 124 | 6.2 | 9.5 | 84 | 0.016 | 8.0 |

Based on SDS-PAGE separation procedures the purified IFN-$\gamma$ is segmented into 4 bands. The average bands are at 20,000 daltons, 25,000 daltons, 47,000 daltons and 63,000 daltons, ± 3,000 daltons. A sample of puri- fied IFN-δ prepared by a process such as in Example 6 was passed through a denaturing gel system to separate the IFN-δ into bands; the bands were then cut out and confirmed for homogeneity. These bands were subject to an amino acid analysis.

The amino acid analysis was run on a 20,000 dalton band, two 25,000 dalton bands (one band had a molecular weight of 25,000 daltons and the other band had a molecular weight of 28,000 daltons), a 45,000 dalton band and a 66,000 dalton band. The individual protein bands were hydrolyzed into their separate amino acids. Each band was then run through a Beckman Amino Acid Analyzer which separated the amino acids and quantitatively measured the amino acid residues per protein. Comparisons of the ratios of the relative peak heights between amino acids for the 25,000, 28,000, 45,000 and 66,000 dalton bands were sufficiently similar to conclude that these bands represent the same protein, and that the 45,000 dalton band is a dimer and the 66,000 dalton band is a trimer of the 25,000 dalton band.

Comparisons of the ratios of the relative peak heights between amino acids for the 20,000 dalton band and the 25,000 dalton band were sufficiently different to conclude the 20,000 dalton band represents a different species of IFN-ᵟ.

The purified IFN-ᵟ is shown to consist of two homogeneous species, namely IFN-ᵟ$_1$ (MW 25,000 daltons and its dimer and trimer), and IFN-ᵟ$_2$ (MW 20,000 daltons.

The following Table III shows the number of amino acid residues per IFN-ᵟ protein molecule. This Table is based on the results of a run through the amino acid analyser for each band.

### TABLE III

#### Number of Residues

| Amino Acids | 25,000 Daltons | 28,000 Daltons | 45,0000 Daltons * | 66,000 Daltons |
|---|---|---|---|---|
| Aspartic Acid | 23.5 | 29.0 | 77 | 55 |
| Threonine | 17.7 | 9.7 | 84** | 32 |
| Serine | 30.7 | 29.5 | | 68 |
| Glutamic Acid | 36.0 | 29.7 | 83 | 93 |
| Proline | 5.4 | 7.7 | – | 21 |
| Glycine | 38.3 | 42.4 | 127 | 106 |
| Alanine | 24.3 | 17.2 | 71 | 64 |
| Valine | 10.6 | 8.4 | – | 27 |
| Isoleucine | 2.8 | 5.2 | – | 18 |
| Leucine | 12.9 | 19.4 | 26 | 38 |
| Phenylalanine | 5.3 | 16.0 | 21 | 15 |

| Amino Acids | 25,000 Daltons | 28,000 Daltons | 45,000 Daltons* | 66,000 Daltons |
|---|---|---|---|---|
| Histidine | 2.2 | - | 5 | 110 |
| Lysine | 9.6 | 15.6 | 30 | 30 |
| Arginine | 7.6 | 23.3 | - | 19 |
| Methionine | - | - | - | 5 |
| Cysteine | - | - | - | - |
| Tyrosine | - | - | - | - |
| Tryptophan | - | - | - | - |

## EXAMPLE 8

### Isolation of IFN-ठ mRNA

The cells, which remained after the supernatant containing the crude IFN-ठ of Example 4 was separated, were used in this Example.

The cells were centrifuged in a Ficoll Hypaque density gradient to isolate viable lymphocytes. The lymphocytes were lysed by dissolving them in 5-10 times their volume in a solution of 4.2 M guanidine thiocyanate, 25 mM sodium citrate, pH 7.0, 0.1 M 2-mercaptoethanol, 0.5% sarkosyl and 0.1% antifoam A. The solution was then homogenized on a sonic blender for one minute to reduce

---

\*  The high numbers for the 45,000 dalton bands were caused by an elevated baseline.

\*\* This represents the total number of residues for Threonine and Serine.

the viscosity by breaking up DNA strands.  Twenty ml. of the solution was layered over 15 ml. of 5.7 M cesium chloride in 0.01 M EDTA in an SW27 cellulose nitrate centrifuge tube, and centrifuged for 72 hours at 22,000 rpm at 15°C.  After aspirating the guanidine thiocyanate solution and decanting the cesium chloride solution, the remaining RNA pellet was rinsed in 500 ml. of cold 70% ethanol to remove excess salt.  The mRNA was dissolved in a solution of 0.01 M tris-HCl, pH 7.4 and 0.001 M EDTA, and then precipitated overnight by making the solution 70% ethanol.  The ethanol was decanted and the remaining RNA pellet air dried and suspended in a salt buffer.  The mRNA was bound to a column of poly (U) Sepharose 4B by passing the mRNA over the column in a solution containing 10mM HEPES, pH 7.4, 3mM EDTA, pH 7.4, 0.5% SDS and 300 mM NaCl.  After rinsing the unbound RNA from the column, the poly (A)-containing mRNA was eluted from the column in a solution containing 2.5 mM EDTA, pH 7.4, 1.0 mM HEPES, pH 7.4 and 70% deionized formamide maintained at 55-65°C.  The mRNA was precipitated in 70% ethanol  twice  and dissolved in 0.01 M tris-HCl, pH 7.4 and 0.001 M EDTA.  The mRNA was then size fractionated over a 5-29.9% isokinetic sucrose gradient in an SW41 rotor by centrifuging 27,000 rpm for 17 hours. Twenty-nine fractions were collected from the gradient and samples from selected fractions were assayed for their ability to produce IFN-$\gamma$ in Xenopus laevis oocytes.

IFN-$\gamma$ mRNA activity of selected fractions collected in Example 8, is shown in Table IV.

TABLE IV

| Sample | Units of IFN-$\gamma$ per mg. mRNA | Units of IFN-$\gamma$ in Sample |
|---|---|---|
| Total mRNA[a] | 61 | 180.0 |
| Sucrose Gradients: | | |
| Fraction 11 | 2,560 | 3.0 |
| Fraction 12 | 240 | 3.1 |
| Fraction 13 | >>2,560[b] | >>3.3 |

[a] mRNA refers to material eluting from the poly (U) Sepharose 4B column.

[b] Protection from cytopathic effects of viral replication was provided at all dilutions of oocyte extract tested.

### Construction and Selection of Transformant

Single-stranded cDNA was transcribed from the above #11 Fraction in the presence of avian myeloblastosis virus reverse transcriptase and a four-fold weight excess of oligo(dT) primer to mRNA. Double stranded cDNA was transcribed from an ss-cDNA template with reverse transcriptase. The ds-cDNA was made blunt-ended by in-

cubation with $S_1$ nuclease. The reaction mixture consisted of 0.2 M sodium acetate pH 4.5, 0.4 M sodium chloride, 2.5 mM zinc sulfate and 1-5 units of $S_1$ nuclease per ng. of ds-cDNA in a final reaction volume of 100 $\mu$l. The ds-cDNA was incubated at 37°C. for 1 hr., extracted with phenol:chloroform, ethanol precipitated and then subjected to size fractionation by electrophoresis on an 8% polyacrylamide gel.

Reaction products with a length of 300 base pairs or longer were eluted, dialyzed against $H_2O$ and lyophilized. Terminal transferase was employed to add approximate 30 dCMP residues to the ds-cDNA 3'-termini.

15 residues of dCMP were added in a similar reaction to the plasmid pBR322 that had been previously cleaved by PstI restriction endonuclease. Equimolar amounts of dC-tailed ds-cDNA and dG-tailed plasmid were annealed in 150 mM NaCl, 10 mM tris-HCl pH 7.6 and 1 mM EDTA at a final DNA concentration of 10 $\mu$g./ml. The annealing mixture was placed at 75°C. and the temperature was lowered to 0°C. over a 5 hr. time period. Transformation of E. coli RR1 was carried out in the presence of calcium chloride. Transformants were selected by growth on tetracycline.

Transformants Screened for Ability to Express IFN-$\gamma$

Transformants were inoculated into individual wells of 96-well microtiter plates, grown to mid-log phase, adjusted to 8% dimethyl sulfoxide, and then maintained as frozen stocks at -70°C. Transformants were "screened" by growing the bacteria on replicate nitrocellulose filters using a 96-prong inoculating apparatus.

Filters were hybridized with $^{32}$P-labeled ss-cDNAs synthesized from mRNA isolated from normal and IFN-δ induced human buffy coat cells. The filters were then washed, dried, and autoradiographed. Colonies that showed a strong hybridization signal with induced cDNA and a weak hybridization signal with normal cDNA were selected for further analysis. Plasmid DNA was isolated, cleaved with restriction endonuclease <u>PstI</u> and the cleavage fragments separated by electrophoresis in horizontal 1.5% agarose gels. Plasmids containing large ds-cDNA inserts were selected for further analysis.

To further confirm the identity of the candidate IFN-δ plasmids, a partial nucleotide sequence of the ds-cDNA insert was determined. DNA inserts are labeled at the 3'-end with $^{32}$P-cordecypin and then subjected to DNA sequence analysis.

## Expression of IFN-δ

High-level expression of IFN-δ in bacterial cells is obtained when the ds-cDNA is cloned in a generalized expression vector which contains the <u>E. coli</u> trp

promoter operator region. Additional expression vectors used include pBR322 vectors into which a synthetic ribosome binding site has been inserted into the $\beta$-lactamase gene.

High level of expression of IFN-$\delta$ in yeast is obtained when the ds-cDNA is inserted into an appropriate site in the yeast 2 u circle to bring expression of IFN-$\delta$ directly under the control of the 2 u promotion function. The IFN-$\delta$ ds-cDNA are also inserted into yeast DNA in such a fashion that expression of the IFN-$\delta$ gene comes under the control of a derepressible yeast enzyme such as acid phosphate gene, pho E. High levels of expression of interferon protein are obtained by inserting this entire construction into the free replicating yeast 2 u circle.

Expression of IFN-$\delta$ in mammalian cells is obtained when the ds-cDNA insert is placed under the control of the SV40 early and late promoters. The recombinant DNA is used to transfect mammalian cells in order to obtain appropriate post-translational modification.

While the invention has been described in terms of preferred embodiments constituting the best mode known to the applicants at the time of this application, various changes may be made in the invention without departing from the scope thereof, which is defined by the following claims.

CLAIMS

1.      A process for the production of IFN-$\gamma$ or its
mRNA comprising incubating a viable cell suspension
for a period of time at least sufficient for the
production of IFN-$\gamma$, characterised in that said
viable cell suspension includes (a) leukocytes,
(b) an inducer for production of IFN-$\gamma$, and
(c) a modulator for said inducer selected from
mezerein and 12,13-phorbol butyrate, said inducer
and said modulator being present in said suspension
in amounts sufficient to stimulate production of
IFN-$\gamma$.

2.      The process of Claim 1, wherein said inducer
is a calcium ionophore selected from A-23187,
2,2-dimethyl-6,6,7,7,8,8,8-heptafluoro-3,5-octanedione
(FOD), and ionomycin.

3.      The process according to Claim 1 or 2, wherein
said leukocytes comprise human peripheral blood
leukocytes.

4.      The process according to Claim 3, wherein,
prior to said incubating step, said human leukocytes
are suspended in a culture medium.

5.      A process according to Claim 4, wherein said
human leukocytes are suspended in a culture medium
in a proportion of from $1.0 \times 10^6$ to $9.0 \times 10^6$
cells/ml. of cell suspension.

6.    The process according to Claim 4, wherein said leukocytes are contained in buffy coats.

7.    The process according to Claim 6, wherein, prior to said suspending step, erythrocytes are separated from said buffy coats.

8.    A process according to Claim 4, wherein said modulator is mezerein, and is present in a minimum proportion of 0.7 ng./ml. of cell suspension.

9.    A process according to Claim 8, wherein the mezerein is present in a proportion of 7-30 ng./ml. of cell suspension.

10.    A process according to Claim 4, wherein said modulator is 12, 13-phorbol dibutyrate, and is present in a minimum proportion of 0.7 ng./ml. of cell suspension.

11.    A process according to Claim 10, wherein the 12,13-phorbol dibutyrate is present in a proportion of 7-30 ng./ml. of cell suspension.

12.    The process according to Claim 4, wherein said inducer is calcium ionophore A-23187, and is present in a proportion of 0.10-10.0 µg./ml. of cell suspension.

13.    The process according to Claim 12, wherein the calcium ionophore A-23187 is present in a proportion of 0.10-2.0 µg./ml. of cell suspension.

14. The process according to Claim 4, wherein said inducer is calcium ionophere FOD and is present in a proportion of O.10-300 µg./ml. of cell suspension.

15. The process according to Claim 4, wherein said incubation period is at least 12 hours.

16. The process according to Claim 15, wherein said incubation period is 3-4 days.

17. The process according to Claim 4, wherein said cell suspension is incubated under relatively stationary conditions.

18. The process according to Claim 17, wherein said inducer is calcium ionophore A-23187 and is present in a proportion of O.15-O.8 µg./ml. of cell suspension.

19. The process according to Claim 18, wherein the calcium ionophore A-23187 is present in a proportion of O.25-O.6 µg./ml. of cell suspension.

20. The process according to Claim 4, wherein said cell suspension is agitated during incubation.

21. The process according to Claim 4, wherein subsequent to said incubation, the supernatant from said cell suspension, which contains IFN-$\gamma$, is separated from the cells.

22.     The process according to Claim 21, including the step of purifying said IFN-ɣ.

23.     A process according to Claim 22, which comprises:

        (a)     adsorbing said IFN-ɣ onto a column containing Controlled Pore Gas beads and eluting with ammonium sulfate;

        (b)     adsorbing said IFN-ɣ containing eluate of step (a) onto a column containing Concanavalin A-Sepharose, lentil lectin-Sepharose or pea lectin-agarose and eluting with a buffer containing a sugar;

        (c)     adsorbing said IFN-ɣ containing eluate of step (b) onto a column containing Heparin-Sepharose or Procian Red-agarose and eluting with a buffer containing a high salt content; and

        (d)     passing the IFN-ɣ containing eluate of step (c) through a gel-filtration column equilibrate in high salt and recovering purified IFN-ɣ.

24.     A process according to Claim 22, which comprises:

        (a) (i)     contacting said IFN-ɣ with Controll:i Pore Glass beads for a period of time sufficient to absorb said IFN-ɣ onto said beads in the presence of a neutral pH buffer;

            (ii)    washing said beads with a chemicall/ compatible buffer until the optical density of the eluant at 280 nm is O;

            (iii)   washing said beads with a neutrə. pH buffer solution until the optical density of ə eluate at 280 nm is O; and

(iv) eluting IFN-ɣ from said beads with an ammonium sulfate solution;

(b) (i) contacting said eluate of step (a)(iv) with an adsorbent selected from the group consisting of Concanavalin A-Sepharose, lentil lectin-Sepharose and pea lectin-agarose for a period of time sufficient to adsorb said IFN-ɣ onto said adsorbent in the presence of a neutral pH buffer;

(ii) washing said adsorbent with ammonium sulfate until the optical density of the eluant at 280 nm is O;

(iii) washing said adsorbent with a neutral pH buffer until the optical density of the eluate at 280 nm is O; and

(iv) eluting IFN-ɣ from said adsorbent with a buffer containing a sugar of the group consisting of alpha-methyl D-mannoside and 1-methyl D-glucoside;

(c) (i) contacting said eluate of step (b)(iv) with an adsorbent selected from the group consisting of Heparin-Sepharose and Procian Red-agarose for a period of time sufficient to adsorb said IFN-ɣ onto said adsorbent in the presence of a neutral pH buffer;

(ii) washing said adsorbent with a neutral pH buffer containing a sugar selected from the group consisting of alpha-methyl D-mannoside and 1-methyl D-glucoside until the optical density of the eluate at 280 nm is O;

(iii) washing said adsorbent with a neutral pH buffer until the optical density of the eluate is O; and,

(iv) eluting IFN-ɣ from said adsorbent with a neutral pH buffer containing a high concentration of salt; and

(d) (i) passing said eluate of step (c)(iv) through a gel-filtration column equilibrated in a neutral pH buffer containing a high concentration of salt; and,

(ii) eluting IFN-ɣ from said column with a neutral pH buffer.

25. The process of Claim 24, wherein homogeneous IFN-ɣ is recovered from the eluate of step (d)(ii).

26. The process according to Claim 24, wherein step (b) is carried out prior to step (a).

27. The process according to Claim 24, wherein the sequence of steps is from step (c) to step (b) to step (a) to step (d).

28. The process according to Claim 24, wherein said IFN-ɣ in said supernatant is produced from cell suspensions containing unlysed red blood cells, and, prior to beginning said purification steps, said supernatant has added thereto sufficient tris-(hydroxymethyl)-aminomethane to bring the concentration to 500 mM, and is buffered to pH 9.5 to 9.7.

29. The process according to Claim 24, wherein the final elution IFN-ɣ is further contacted with phenyl-Sepharose for a period of time sufficient to accomplish equilibrated adsorption of said IFN-ɣ to the phenyl-Sepharose, and the adsorbed IFN-ɣ is eluted with low salt buffers.

30.    The process according to Claim 22, which comprises:

(a) contacting said IFN-ɤ with a resin selected from Heparin-Sepharose and Procian-Red agarose for a period of time sufficient to accomplish equilibrated adsorption of said IFN-ɤ on to the resin;

(b) washing said resin with phosphate buffered saline solution containing a sugar selected from O.1M to 2M alpha-methyl D-mannoside and 1-methyl D-glucoside until the optical density at 280 nm is O;

(c) washing with phosphate buffered saline solution until the optical density at 280 nm is O;

(d) eluting the adsorbed IFN-ɤ with phosphate buffered saline containing 2M sodium chloride; and

(e) recovering substantially pure IFN-ɤ.

31.    A process according to Claim 22, which comprises:

(a) contacting said IFN-ɤ with Controlled Pore Glass beads for a period of time sufficient to adsorb said IFN-ɤ on to said beads in the presence of a neutral pH buffer;

(b) washing said beads with a chemically compatible buffer until the optical density of the eluant at 280 nm is O;

(c) washing said beads with a neutral pH buffered solution until the optical density of the eluant at 280 nm is O;

(d) eluting IFN-ɤ from said beads with an ammonium sulfate solution; and

(e) recovering substantially pure IFN-ɤ.

32.    The process according to Claim 21, wherein
IFN-ɣ mRNA is extracted from said separated cells.

33.    The process according to Claim 32, wherein
said IFN-ɣ mRNA is extracted by the steps, comprising:

    (a)    Lysing the cells and removing large
contaminants;

    (b)    precipitating remaining RNA in alcohol
and then resuspending in a salt buffer;

    (c)    passing the RNA suspension through a
poly-(U) column;

    (d)    eluting the mRNA from said column; and

    (e)    size fractionating the eluted mRNA.

34.    The process according to Claim 33, wherein
said IFN-ɣ mRNA is extracted by the steps comprising:

    (a)    lysing said cells in a solution comprising
sodium acetate and SDS, or guanidine thiocyanate;

    (b)    removing large strands of DNA and protein
by phenol extraction or centrifuging with
cesium chloride in a cellulose nitrate tube;

    (c)    precipitating the remaining mRNA in
ethanol and resuspending in a salt buffer;

    (d)    passing the mRNA suspension through a
poly-(U) Sepharose-4B column and washing with
a buffer;

    (e)    eluting the mRNA from said colum with
formamide; and

    (f)    size fractionating the eluted mRNA in
sucrose gradient.

35.    The process according to Claim 34, wherein samples of the fractions of extracted IFN-ɣ mRNA are inserted into cells capable of expressing IFN-ɣ, said cells are allowed to express said IFN-ɣ, and quantitative expression of IFN-ɣ by said cells is measured.

36.    A process for producing clones that express IFN-ɣ comprising:

(a)    adding reverse transcriptase to IFN-ɣ mRNA from high IFN-ɣ activity fractions obtained in accordance with Claim 35, to transcribe the single-stranded complementary IFN-ɣ DNA;

(b)    adding DNA polymerase or reverse transcriptase to said single-strand DNA to produce double-stranded IFN-ɣ DNA;

(c)    making the IFN-ɣ ds-DNA blunt-ended by incubation with nuclease;

(d)    selected a cloning vehicle carrying a phenotypical trait, said vehicle being compatible with a host cell;

(e)    cleaving said cloning vehicle in the presence of restrictive endonuclease;

(f)    combining said cleaved vehicle with said IFN-ɣ double-stranded DNA in the presence of DNA ligase to form a recombinant vehicle;

(g)    adding complementary terminal codons to the blunt-ended IFN-ɣ ds-DNA and the cleaved cloning vehicle in the presence of terminal transferase;

(h)    transforming the host cell with the recombinant vehicle in the presence of calcium chloride; and

(i) growing said host cells and then isolating the transformant containig the recombinant vehicles by means of the phenotypical trait.

37. The process for expressing IFN-$\gamma$ comprising:

(a) selecting the transformants of step (i) of claim 36 which demonstrate strong hybridization to mRNA from IFN-$\gamma$ induced cells; and

(b) removing IFN-$\gamma$ ds-DNA from said transformants and inserting it in an optimal expression cloning vehicle.

38. Homogeneous IFN-$\gamma$.

39. Homogeneous human IFN-$\gamma$.

40. The IFN-$\gamma_1$ species of said homogeneous human IFN-$\gamma$ of Claim 39 corresponding to an SDS-PAGE molecular weight band of 25,000 $\pm$ 3,000 daltons.

41. The dimer of the IFN-$\gamma_1$ species of said IFN-$\gamma$ of Claim 39 corresponding to an SDS-PAGE molecular weight band of 47,000 $\pm$ 3,000 daltons.

42. The trimer of the IFN-$\gamma_1$ species of said IFN-$\gamma$ of Claim 39 corresponding to an SDS-PAGE molecular weight band of 63,000 $\pm$ 3,000 daltons.

43. The IFN-$\gamma_2$ species of said homogeneous human IFN-$\gamma$ of Claim 39 corresponding to an SDS-PAGE molecular weight band of 20,000 $\pm$ 3,000 daltons.

44. The recombinant cloning vehicle containing DNA segment coding for human IFN-$\gamma$.

45. The host cell containing the recombinant cloning vehicle of Claim 44.

FIG. 1 (Sheet 1)

BLOOD → CENTRIFUGE → PLASMA

CENTRIFUGE → BUFFY COATS → REMOVE RED BLOOD CELLS → LEUKOCYTES

BUFFY COATS → SUSPEND IN CULTURE MEDIUM CONTAINING ANTIBIOTICS

ADMIX MODULATOR AND INDUCER → SUSPEND IN CULTURE MEDIUM CONTAINING ANTIBIOTICS

SUSPEND IN CULTURE MEDIUM CONTAINING ANTIBIOTICS → CONDITION → ADD PROTEIN SOURCE → INCUBATE STATIONARY / INCUBATE STIR → CENTRIFUGE

CENTRIFUGE → SUPERNATANT (CRUDE IFN-γ) → PURIFY → HOMOGENEOUS IFN-γ → SPECIES 1 / SPECIES 2

CENTRIFUGE → CELLS CONTAINING m-RNA → LYSE CELLS → EXTRACT LARGE DNA STRANDS AND PROTEIN → PASS THROUGH POLY (U) COLUMN AND ELUTE m-RNA → SIZE FRACTIONATE ELUATE → SAMPLES FROM FRACTIONS ARE ASSAYED FOR ABILITY TO PRODUCE IFN-γ IN FROG OOCYTES → FRACTIONS CONTAINING m-RNA WITH HIGH IFN-γ ACTIVITY

TO FIG. 1 SHEET 2

FIG. 1 SH. 1 / FIG. 1 SH. 2

FIG. 1

FROM
FIG. I
SHEET I

ADD
REVERSE
TRANS-
CRIPTASE

ss-DNA

ADD DNA
POLYMERASE
OR REVERSE
TRANSCRIPTASE

ds-DNA

BLUNT ENDS
WITH
NUCLEASE

ADD TERMINAL
CODONS WITH
TERMINAL
TRANSFERASE

CLEAVE WITH
RESTRICTIVE
ENDONUCLEASE

CLONING
VEHICLE
WITH
PENOTYPICAL
TRAIT

CLEAVED
CLONING
VEHICLE

ADD TERMINAL
CODONS WITH
TERMINAL
TRANSFERASE

JOIN IN
PRESENCE OF
DNA LIGASE

RECOMB-
INANT
CLONING
VEHICLE

HOST
CELL

TRANSFORM IN
PRESENCE OF
CALCIUM
CHLORIDE

ISOLATE
TRANSFORMANT
BY PHENO-
TYPICAL TRAIT

SELECT
TRANSFORMANTS
WITH STRONG
HYBRIDIZATION
TO LABELED
m-RNA FROM
INDUCED CELLS

TEST FOR
EXPRESSION

INSERT IN
OPTIMAL
EXPRESSION
VEHICLES FOR
LARGE SCALE
PRODUCTION

_FIG. I_
(Sheet 2)

2/3

0063482

INTERFERON (X10³ UNITS/ml.)

A-23187

DOSE RESPONSE OF A-23187 (ug./ml.)
IN 70 ng./ml. OF MEZEREIN.

FIG. 2.

MEZEREIN

DOSE RESPONSE OF MEZEREIN (ng./ml.)
IN .5 ug./ml. OF A-23187.

FIG. 3

3/3

0063482